# EUROPEAN PATENT APPLICATION

(11) **EP 2 377 852 A1**
(43) Date of publication of application: **19.10.2011**
(21) Application number: 10305372.4
(22) Date of filing: 09.04.2010
(51) Int. Cl.: C07D 221/12, C07D 401/12, C07D 401/14, A61K 31/473, A61P 25/28

(54) **Labelled huprine derivatives and their use in medical imaging**

(71) Applicant: Universite de Rouen, 76821 Mont-Saint-Aignan Cédex (FR); Advanced Accelerator Applications, 01630 Saint Genis Pouilly (FR)
(72) Inventor: Bouteillier, Cédric, 01630, Saint-Genis-Pouilly (FR); Mariani, Maurizio Franco, 10015, IVREA (To) (IT); Ronco, Cyril, 76130, MONT-SAINT-AIGNAN (FR); Renard, Pierre Yves, 75004, PARIS (FR); Jean, Ludovic, 76000, ROUEN (FR); Romieu, Anthony, 76000, ROUEN (FR)
(74) Representative: Touati, Catherine

(57) **Abstract**

The present invention concerns new Huprine derivatives of formula I: 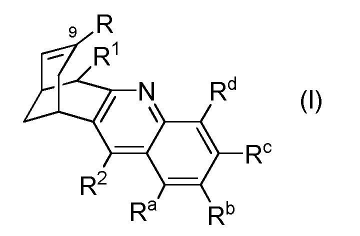 wherein R comprinses a fluorophore or a nuclear probe and their use in medical imaging and in radiotherapy.

## Description

The present invention concerns new Huprine derivatives comprising a fluorophore or a nuclear probe and their use in medical imaging, respectively in optical imaging, MRI or nuclear imaging, and in radiotherapy. Huprine derivatives are acetylcholinesterase inhibitors and, for this reason, labelled Huprines derivatives are useful for investigating the role of such enzyme in the mechanism of neurological diseases in which the level of acetylcholine is affected such as Alzheimer's disease.

Although the cholinergic deficiencies are a wellknown symptom of Alzheimer's disease, their exact role in the first stages of the disease is still controversial. Ability to image acetylcholine activity in human brain *in vivo* is needed to assess directly the involvement of the cholinergic system in neurodegenerative diseases and their current therapies, particularly the Alzheimer's disease. This will help to justify or refute current hypothesis concerning the role of the cholinergic system in the Alzheimer's disease.

Cholinesterases (AChE and BChE, or ChE) are key enzymes ending the process of nerve impulse transmission and their enzymatic activity is directly related to the cholinergic activity. Cholinesterases (ChE) inhibitors are commonly used in palliative treatment of neurological diseases in which the level of acetylcholine is affected such as Alzheimer's disease. Also, labeled ChE inhibitors are used in medical imaging techniques, such as positron emission tomography (PET), for monitoring cholinergic activity. Such techniques can detect the decrease of enzymatic activity induced by a neurological disease or measure the inhibiting efficiency of the ChE inhibitors. Cerebral ChE imaging is useful for diagnosis of dementia disorders and also for therapeutic monitoring of the effects of ChE inhibitors and for decision of the appropriate clinical dosage of newly developed ChE inhibitors. Several ChE inhibitors or derivatives thereof have been labeled with positron emitters for mapping cerebral ChE by positron emission tomography (PET). However, the available radiolabeled ChE inhobitors, such as ¹¹C-physostigmine and 11C-donepezil, do not exhibit enough strong signals and poorly reflect the regional distribution of ChE in the brain because of high non-specific binding and/or less specific to ChE *in vivo* in the brain tissue. Fluorinated Huprine derivatives were described (J. Med. Chem. 1999, 42 3227-3242). However, the route for synthetising such products do not allow a quick introduction of the fluorine atom in the Huprine structure, which is necessary especially for short time half-life radioisotope ¹⁸F used for PET. Also, the chemical structures of the compounds approved by international regulatory organizations regarding public health (Tacrine (Cognex ®), Donepezil (E2020, Aricept®), Rivastigmine (Exlon®) and Galanthamine (Reminyl®)) are not easily functionalizable and are not suitable for use as precursors for preparing labelled ChE inhibitors.

Therefore there is a need to provide new and more specific cholinesterase (AChE or BChE) inhibitors, able to be labelled for medical imaging techniques, to cross the blood brain barrier and to have a specific and efficient brain mapping of AChE or BChE distribution. Such compounds would also be useful for monitoring the evolution of the AChE or BChE distribution during Alzheimer's disease progress and evaluate the efficiency of the palliative treatments.

European patent application EP 10305366.6 describes new highly functionalizable Huprine derivatives. The high functionalizability of these compounds allows introducing any desired label. In particular, by choosing the appropriate function, it is possible to quickly introduce the desired label which is of great interest for short time half-life radioisotopes such as ¹⁸F.

Therefore the present invention provides new Huprine derivatives comprising a fluorophore or/and a nuclear probe. Such labelled compounds are useful for monitoring, analysing and measuring the AChE or BChE enzymatic activity with medical imaging techniques.

The present invention thus concerns labelled Huprine derivatives of formula I : 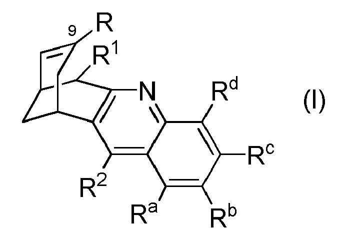 wherein,
R is a moiety comprising a fluorophore or a nuclear probe;
R^{a}, R^{b}, R^{C} and R^{d} are independently H, halogen, cyano, carboxyl, ―O(C1―C₄ alkyl), ―(C₁―C₄ alkyl), or ―CH₂S(C₁-C₄ alkyl); preferably H, halogen, cyano, ―SCH₃ or -CH₂SCH₃; more preferably H or Cl; even more preferably R^{a}, R^{b} and R^{d} are H and R^{C} is H or Cl;
R¹ is H or =CH₂; and
R² iₛ Cl or NR³R'³ wherein R³ and R'3 are independently H, acetyl, C₁-C₄ alkyl, —CO(C₁-C₄ alkyl) or any hydrocarbyl chain linked to a ligand of the peripheral site of the AChE or BChE, said ligand being preferably selected from phenyltetraisoquinoline derivatives, beta-carboline derivatives, indolic derivatives or coumarine derivatives; preferably, R³ and R'3 are independently H, acetyl, C₁-C₄ alkyl or ―CO(C₁-C₄ alkyl); more preferably, R³ and R'³ are independently H or acetyl,
and pharmaceutically acceptable salts thereof.

The expression "C₁-C₄ alkyl" represents any monovalent radical of a linear or branched hydrocarbon chain comprising 1 to 4 carbon atom such as methyl, ethyl, *n*-propyl, *i*-propyl, *n*-butyl, *i*-butyl, *s*-butyl or *t*-butyl.

The term "fluorophore" represents a component of a molecule which causes a molecule to be fluorescent in sufficient quantity and activity to generate a signal in an organism, including a human. It is a functional group in a molecule which will absorb energy of a specific wavelength and re-emit energy at a different wavelength. The fluorophore can be quantum dots, i.e. fluorescent semiconductor nanoparticles, or aromatic or conjugated small molecules which fluoresce thanks to delocalized electrons. The fluorophore is for example selected from fluoresceine derivatives (such as fluoresceine isothiocyanate (FITC)), coumarine derivatives (such as hydroxycoumarin, aminocoumarin and methoxycoumarin), rhodamine derivatives (such as substituted rhodamine isothiocyanate (XRITC)), cyanine derivatives (such as allophycocyanin (APC)) and R-phycoerythrin (PE) or other commercially available fluorescent dyes such as the CF dyes, the FluoProbes dyes, the DyLight Fluors, the Oyester dyes, the Atto dyes, the HiLyte Fluors, and the Alexa Fluors. Preferred fluorophores are selected from rhodamine derivatives and coumarine derivatives.

The term "nuclear probe" means a radioactive or paramagnetic substance or a chelator comprising such a substance attached to the compound in sufficient quantity and activity to generate a signal in an organism, including a human. That is, the nuclear probe forms part of the chemical structure of the inhibitor and comprises a radioactive or non-radioactive isotope present at a level significantly above the natural abundance level of said isotope. Such elevated or enriched levels of isotope are suitably at least 5 times, preferably at least 50 times the natural abundance level of the isotope in question, or present at a level where the level of enrichment of the isotope in question is 90 to 100% of the total quantity of nuclear probe attached. Nuclear probes may include CH₃ groups on the present probes with elevated levels of ¹³C or ¹¹C and fluoroalkyl groups with elevated levels of ¹⁸F, such that the nuclear probe is the isotopically labeled ¹³C, ¹¹C or ¹⁸F within the chemical structure of the probe. Preferred nuclear probes are those which can be detected externally in a non-invasive manner following *administration in vivo.* The nuclear probe is preferably chosen from: (i) a positron emitting radioactive substance; (ii) a gamma-emitting radioactive substance; (iii) a hyperpolarised NMR-active nucleus; (iv) a β⁻-emitting radioactive substance; (v) an α-emitting radioactive substance; and (vi) an Auger electron-emitting substance. Most preferred nuclear probes are radioactive, especially radioactive metal ions, gamma-emitting radioactive halogens and positron-emitting radioactive non-metals, particularly those suitable for imaging using SPECT or PET. The positron-emitting substance is for example a positron-emitting radioisotope among radioactive non-metal, radioactive transition elements plus lanthanides and actinides, and metallic main group elements. Suitable such positron-emitting radioisotopes include ⁶⁴Cu, ⁴⁸V, ⁵²Fe, ⁵⁵Co, ^{94m}Tc, ^{6g}Ga¹¹C, ¹³N, ¹⁵O, ¹⁷F, ¹⁸F, ⁶⁴Cu, ⁶²Cu_{,} ⁷⁵Br, ⁷⁶Br or ¹²⁴I. Preferred positron-emitting radioisotopes ¹¹C ¹³N ¹²⁴I and ¹⁸F, especially ¹¹C and ¹⁸F, most especially ¹⁸F. The gamma-emitting substance can be a gamma-emitting radioisotope among radioactive halogen, radioactive transition elements plus lanthanides and actinides, and metallic main group elements. Suitable gamma-emitting radioisotopes include ^{99m}Tc, ¹¹¹In, ^{113m}In, ⁶⁷Cu, ⁶⁷ Ga, ¹²³I, ¹²⁵I, ¹³¹I or ⁷⁷ Br. Preferred gamma-emitting radioisotope are ¹²⁵I and ^{99m}Tc. The suitable paramagnetic metal ions include Gd (III), Mn (II), Cu (II), Cr (III), Fe (III), Co (II), Er (II), Ni (II), Eu (III) or Dy (III). Preferred paramagnetic metal ions are Gd (III), Mn (II) and Fe (III), with Gd (III) being especially preferred. The hyperpolarised NMR-active nuclei have a non-zero nuclear spin, and include ¹³C, ¹⁵ N_{,} ¹⁹F, ²⁹Si and ³¹P. Of these, ¹³C is preferred. Contrary to other nuclear probes cited above, β⁻-emitting radioactive substances, α-emitting radioactive substances and Auger electron-emitting substances are generally used in radiotherapy rather than in medical imaging technology. Suitable β⁻-emitting radioactive substances include ¹³¹I, ¹⁷⁷Lu, ⁹⁰Y ⁸⁹Sr, ¹⁵³Sm, ¹⁸⁸Re and ¹⁸⁶ Re. Preferred β⁻-emitting radioisotopes is ¹³¹I Advantageously, the α-emitting radioactive substance is seleceted from ¹⁵³Sm ²¹¹At, ²¹²Bi and ²¹²Pb_{.} Suitable Auger electron-emitting substances include In, Gd, Pt, Ru, Os, Au, La, Ce, Ba, Cs, I, Te, Sb, Sn, Cd, Ag and Pd. Of these, In, Gd, Pt, Pd and Au are preferred.

The term "chelator" as used in the present invention refers to a chemical moiety that binds noncovalently to, or complexes with, one or more ions. Chelators can bind to lithium, calcium, sodium, magnesium, potassium, and/or other biologically important metal ions, although in the present case it binds to a radioisotope or paramagnetic metal ion. Examples of chelators are well known in the art. Preferably, the chelator binds a metal cation. Suitable chelators are bipyridyl (bipy); terpyridyl (terpy); ethylenediaminetetraacetic acid (EDTA); crown ethers; aza-crown ethers; succinic acid; citric acid; salicylic acids; histidines; imidazoles; ethyleneglycol-bis-(beta-aminoethyl ether) N,N'-tetraacetic acid (EGTA); nitroloacetic acid; acetylacetonate (acac); sulfate; dithiocarbamates; carboxylates; alkyldiamines; ethylenediamine (en); diethylenetriamine (dien); nitrate; nitro; nitroso; (C₆ Hs)₂ PCH₂ CH₂ P(C₆ H5)₂ (diphos); glyme; diglyme; bis (acetylacetonate) ethylenediamine (acacen); 1,4,7,10-tetraazacyclododecanetetraacetic acid (DOTA), 1,4,7,10- tetraazacyclododecane-1,4,7-triacetic acid (D03A), 1-oxa-4,7,10-triazacyclododecane- triacetic acid (OTTA), 1,4,7-triazacyclononanetriacetic acid (NOTA), 1,4,8,11-tetraazacyclotetradecanetetraacetic acid (TETA), DOTA-N-(2-aminoethyl) amide; DOTA-N- (2-aminophenethyl) amide; and 1,4,8,11-tetraazacyclotetradecane.

In a first embodiment, R comprises a fluorophore.

In a second embodiment, R comprises a nuclear probe.

In a particular embodiment, R comprises a nuclear probe and a fluorophore. In another embodiment, R comprises two nuclear probes, for example a positron-emitting substance and a β⁻-emitting substance or a gamma-emitting subtancce and a β⁻-emitting substance.

Advantageouly, R can further comprise a lipophilic vector.

The term "lipohilic vector" in the present invention refers to any moiety that modifies the lipophilic affinity of the compound of the invention to allow crossing the blood-brain barrier (BBB). Such definition includes brain targeting chemical delivery systems (BTCDS). BTCDS refers to chemical compounds covalently bound to the active compound. BTCDS are well known in the art *(*The Open Medicinal Chemistry Journal, 2008, 2, 97-100; J. Med. Chem. 1996, 39, 4775-4782). Any suitable BTCDS, such as 1,4-dihydrotrigonellyl/trigonelline based systems, can be used in the compounds of the invention. The BTCDS modifies the lipophilic affinity of the compound of invention to allow crossing the BBB and is enzymatically hydrolysable once in the brain. For this purpose, the BTCDS is advantageously composed of a lipophilic transport moiety and conveniently choosen enzyme labile spacer. The lipophilic transport moiety is for example selected from 1,4-dihydrotrigonellyl, esters of highly lipophilic alcohol such as cholesterol. The enzyme labile spacer is for example an aminoacid spacer strategically selected to ensure the release of the labelled AChE or BChE inhibitor through specific peptidase once in the brain. Suitable aminoacid spacers are for example described by Prokai and Bodor (Science, 1992, 257, 1698; N. J. Med. Chem., 1996, 39, 4775; and Open Med. Chem. J., 2008, 2, 97-100,). Preferred spacers are for example the proline (PRO) or alanine (ALA) spacers.

Preferably, R is a moiety of formula:

-X-Y-Z

wherein,
X is C₁-C₆ alkylene or C₂-C₆ alkenylene; in a preferred embodiment, X is a linear C₁-C₆ alkylene or a linear C₂-C₆ alkenylene; more preferably, X is a linear C₁-C₆ alkylene; even more preferably, X is a a linear C₃-C₆ alkylene; in another preferred embodiment, X is a branched C₁-C₆ alkylene or a branched C₂-C₆ alkenylene;
Y is a single bound, ―O―, ―C(O)―, ―C(O)O―, ―OC(O)―, ―NH―N=C―, ―O―N=C―, ―C=N―NH―, ―C=N―O―, ―CH(Z')―, ―CH(CH₂Z')―CH₂―, ―N(Z')― 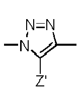 or 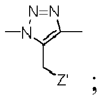
Z is a fluorophore or a nuclear probe; and
Z' is H, a lipophilic vector, a fluorophore or a nuclear probe.

The expression "C₁-C₆ alkylene" represents any divalent radical of a linear or branched hydrocarbon chain comprising 1 to 6 carbon atoms, such as ―CH₂―, ―(CH₂)₂―, ―(CH₂)₃-, ―(CH₂)₄―, ―(CH₂)₅―, ―(CH₂)₆―, ―CH(CH₃)―CH₂―, ―CH₂―CH(CH₃)―, ―CH₂―CH₂―CH(CH₃)―, ―CH₂―CH(CH₃)―CH₂― or ―CH(CH₃)―CH₂―CH₂―.

The expression "C₂-C₆ alkenylene" represents any divalent radical of a linear or branched hydrocarbon chain comprising 2 to 6 carbon atoms and at least one double bound, such as ―CH=CH―, ―CH=CH―CH₂―, ―CH₂―CH=CH― ―CH=CH―CH₂―CH₂―, ―CH₂―CH₂―CH=CH― or ―CH₂― CH=CH―CH₂―

More preferably, R is selected from the group consisting of -(CH₂)₂-Z, ―(CH₂)₃―Z, ―(CH₂)₄―Z, ―(CH₂)₅―Z, ―(CH₂)₆―Z, ―(CH₂)₂―CH(Z')―Z, ―(CH₂)₂―CH(CH₂Z')―CH₂―Z, 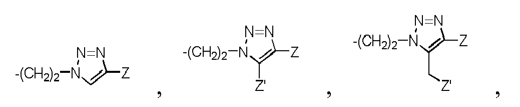 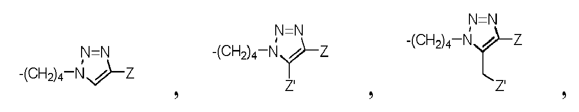 ― (CH₂)₂―NH―N=C―Z, ―(CH₂)₂―O―N=C―Z, ―(CH₂)₂―C=N―NH―Z, ―(CH₂)₂―C=N―O―Z, wherein,
Z is a fluorophore or a nuclear probe; and
Z' is a lipohilic vector, a fluorophore or a nuclear probe.

In a first embodiment, the present invention concerns labelled Huprine derivatives of formula Ia: 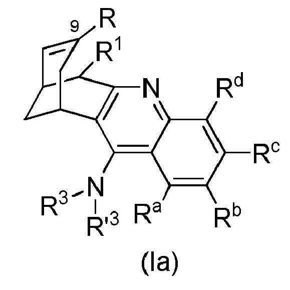 wherein R, R¹, R³, R'3, R^{a}, R^{b}, R^{c} and R^{d} are as defined in formula I; preferably, R^{a}, R^{b} and R^{d} are H and R, R¹, R³, R³ and R^{c} are as defined in formula I;
and pharmaceutically acceptable salts thereof.

In a first alternative of this embodiment, the present invention concerns labelled Huprine derivatives of formula Ia-1: 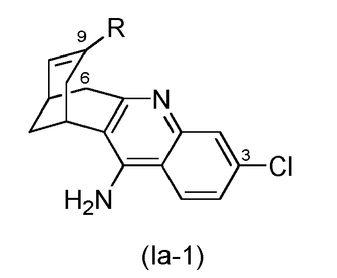 wherein R is as defined in formula I;
and pharmaceutically acceptable salts thereof.

In a second alternative of this embodiment, the present invention concerns labelled Huprine derivatives of formula Ia-2: 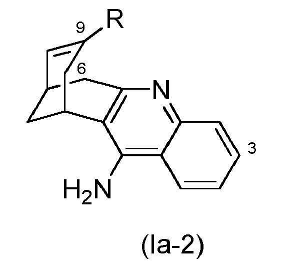 wherein R is as defined in formula I;
and pharmaceutically acceptable salts thereof.

In a third alternative of this embodiment, the present invention concerns labelled Huprine derivatives of formula Ia-3: 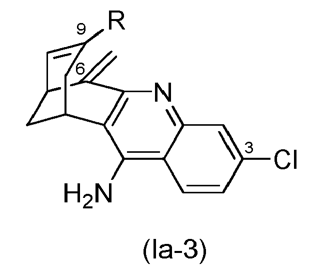 wherein R is as defined in formula I;
and pharmaceutically acceptable salts thereof.

In a second embodiment, the present invention concerns labelled Huprine derivatives of formula Ib: 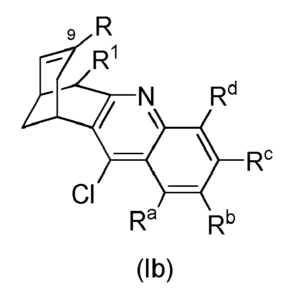 wherein R, R¹, R³, R'³, R^{a}, R^{b}, R^{c} and R^{d} are as defined in formula I; preferably, R^{a}, R^{b} and R^{d} are H and R, R¹, R³, R'³ and R^{c} are as defined in formula I;
and pharmaceutically acceptable salts thereof.

In an alternative of this embodiment, the present invention concerns labelled Huprine derivatives of formula Ib-1: 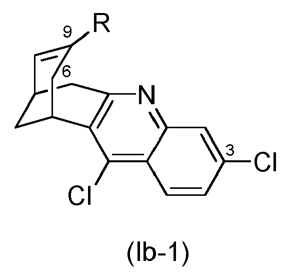 wherein R is as defined in formula I;
and pharmaceutically acceptable salts thereof.

In a particularly preferred embodiment, the compounds of the present invention are selected from the group consisting of: 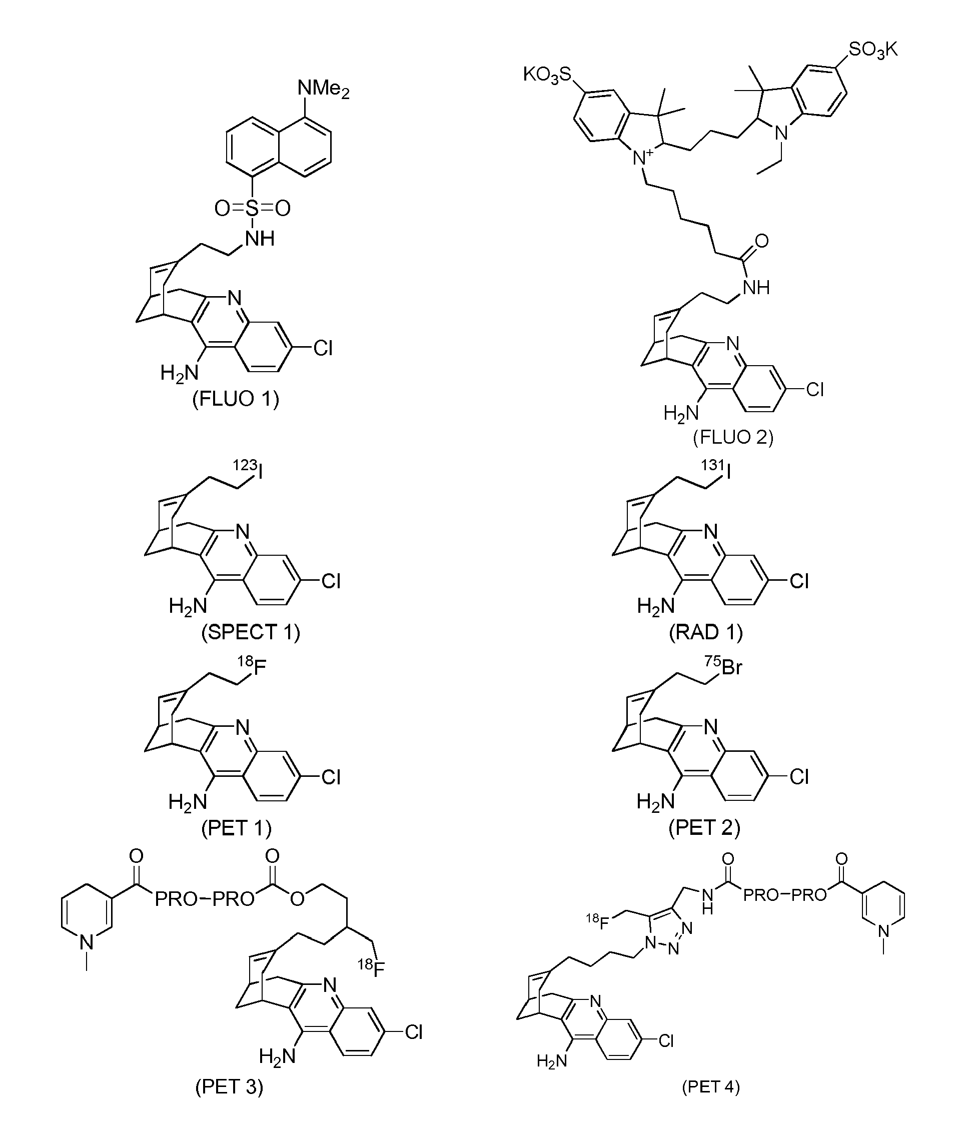 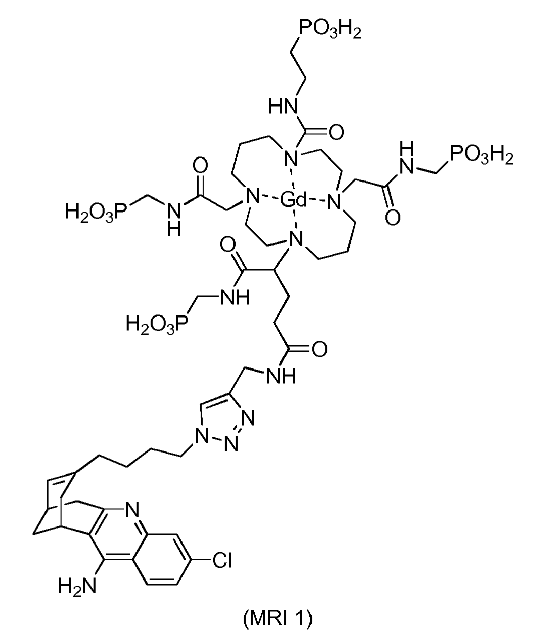 and pharmaceutically acceptable salts thereof.

The compounds of the invention are obtainable from appropriately fonctionalized Huprine derivatives described in European patent application EP 10305366.6. The compounds of the invention are therefore obtainable from a precursor of formula HUP according to the following scheme: 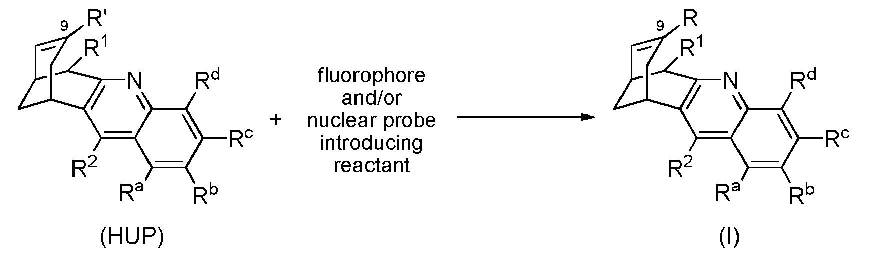 wherein R, R¹, R², R^{a}, R^{b} R^{c} and R^{d} are as defined above and R' is an appropriately functionalized moiety. The synthesis routes for obtaining the compound of formula I from the prescursor of formula HUP comprise common reactions well known to the skilled person. It includes for example nucleophilic substitution, click chemistry reactions or coupling reactions such as between aldehyde/hydrazine or aldehyde/oxamine coupling reactions. The skilled person would be able to determine the adequate precursor and the corresponding synthesis route to introduce the fluorophore or the radiolabelnuclear probe.

The following table illustrates some possible synthesis routes for obtaining the compounds of formula I.

| **Precursor of formula HUP wherein R'is** | **Fluorophore and/or nuclear probe introducing reactant** | **Compound of formula I wherein R is** |
|---|---|---|
| ―(C1-C6 alkylene)―A or (C2-C6 alkenylene)―A wherein A is a leaving group such as ―OMs, ―OTs, ―OTf or ―C(O)NHS | a nucleophilic compound of formula Z⁻ | ―(C1-C6 alkylene)―Z or ― (C2-C6 alkenylene)―Z |
| ―(C1-C6 alkylene)―N₃ | 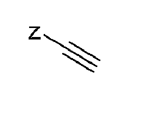 | 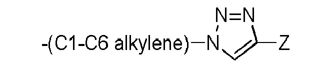 |
| or | | or |
| ―(C1-C6 alkenylene)―N₃ | | 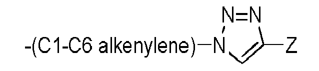 |
| ―(C1-C6 alkylene)―N₃ | 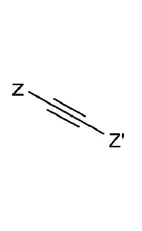 | 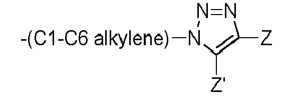 |
| or | | or |
| ―(C1-C6 alkenylene)―N₃ | | 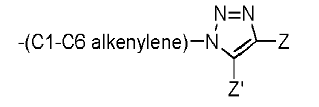 |
| ―(C1-C6 alkylene)―N₃ | 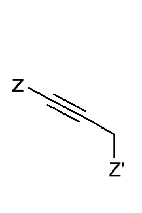 | 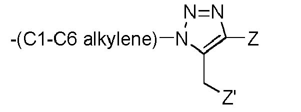 |
| or | | or |
| ―(C1-C6 alkenylene)―N₃ | | 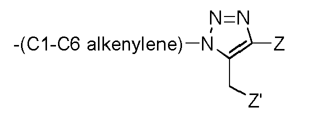 |
| ―(C1-C6 alkylene)―NH-NH₂ or --(C1-C6 alkenylene)―NH-NH₂ | Z―CHO | ―(C1-C6 alkylene)―NH―N=C―Z or ― (C1-C6 alkenylene)―NH―N=C―Z |
| ―(C1-C6 alkylene)―O―NH₂ or ― (C1-C6 alkenylene)―O―NH₂ | Z―CHO | ―(C1-C6 alkylene)―O―N=C―Z or ― (C1-C6 akenylene)―O―N=C―Z |
| ―(C1-C6 alkylene)―CHO or ― (C1-C6 akenylene)―CHO | Z―NH―NH₂ | ―(C1-C6 alkylene)―C=N―NH―Z or ― (C1-C6 alkenylene)―C=N―NH―Z |
| ―(C1-C6 alkylene)―CHO or ― (C1-C6 alkenylene)―CHO | Z―O―NH₂ | ―(C1-C6 alkylene)―C=N―O―Z or ― (C1-C6 alkenylene)―C=N―O―Z |

The compounds of formula I are inhibitors of AChE or BChE and therefore bind to AChE or BChE. These compounds may thus be useful for treatment, in vivo diagnosis and imaging of diseases and conditions in which the level of acetylcholine is affected. Such diseases or conditions include neurological conditions such as Alzheimer's disease, multiple sclerosis, cognitive disorders, memory disorders, depressive disorders, bipolar disorders and schizophrenic disorders, Parkinson's disease, Huntington's disease, vascular dementia, fronto-temporal dementia, Lewy bodies dementia, Creutzfeld-Jacob disease, epilepsy, migraine, anxiety, panic, psychosis, hypersensitive syndrome and pain, or cancers. In particular, the compounds of formula I can be used for *treatment, in vivo* diagnosis and imaging of Alzheimer's disease.

Compounds of the invention are provided for use as imaging agent. In particular, the compounds of formula I are provided for use in medical imaging techniques including positron emission tomography (PET), single photon emission computed tomography (SPECT), magnetic resonance imaging (MRI), scintillation or fluorescence imaging. The skilled person can easily choose the appropriate fluorophore or nuclear probe corresponding to the desired imaging technique.

Compounds of formula I wherein R comprises a fluorophore are provided for use *in in vivo or in vitro* fluorescence imaging, for example in fluorescence endoscopic imaging. The fluorophore is for example selected from fluoresceine derivatives (such as fluoresceine isothiocyanate (FITC)), coumarine derivatives (such as hydroxycoumarin, aminocoumarin and methoxycoumarin), rhodamine derivatives (such as substituted rhodamine isothiocyanate (XRITC)), cyanine derivatives (such as allophycocyanin (APC)) and R-phycoerythrin (PE) or other commercially available fluorescent dyes such as the CF dyes, the FluoProbes dyes, the DyLight Fluors, the Oyester dyes, the Atto dyes, the HiLyte Fluors, and the Alexa Fluors. The fluorophore is advantageously selected from cyanine derivatives, dansyl derivatives, coumarine derivatives and rhodamine derivatives. For example, compounds of formulae FLUO1 and FLUO2 can advantageously be used in fluorescence imaging.

Compounds of formula I wherein R comprises a positron-emitting radioisotopes, such as ⁶⁴Cu ⁴⁸V, ⁵²Fe ⁵⁵Co, ^{94m}Tc, ⁶⁸Ga ¹¹C ¹³N_{,} ¹⁵O, ¹⁷F, ¹⁸F, ⁶⁴Cu, ⁶²Cu, ⁷⁵Br, ⁷⁶Br or ¹²⁴I, preferably ¹¹C, ¹³ N, ¹²⁴I and ¹⁸F, and more preferably ¹¹C and ¹⁸F are suitable for use in PET imaging. In particular, the compound of formulae PET1, PET2, PET3 and PET4 can be advantageously used in PET imaging.

Compounds of formula I wherein R comprises a gamma-emitting radioisotopes, such as ^{99m}Tc, ¹¹¹In, ^{113m} In, ⁶⁷Cu, ⁶⁷Ga, ¹²³I, ¹²⁵I, ¹³¹I or ⁷⁷Br, preferably ^{l25}I and ^{99m}Tc are provided for use in SPECT imaging. For example, the compound of formula SPECT1 can be advantageously used in SPECT imaging.

Compounds of formula I wherein R comprises a paramagnetic metal ion, such as Gd (III), Mn (II), Cu (II), Cr (III), Fe (III), Co (II), Er (II), Ni (II), Eu (III) or Dy (III), preferably Gd (III), Mn (II) or Fe (III), or a hyperpolarised NMR-active nucleus, such as ¹³C, _{,}¹⁵N, ¹⁹F, ²⁹Si and ³¹P, preferably ¹³C are suitable for use in MRI imaging. In particular, the compound of formula MRI1 can be advantageously used in MRI imaging.

The present invention also concerns a method for medical imaging, *especially in vivo* imaging, comprising the step of administering to a patient a compound of formula I to a mammal, such as a human, and obtaining an image with an apparatus detecting the radiation emitted by the administered compound. Depending on the labelled compound of formula I to be detected, the skilled person can select the appropriate imaging apparatus.

The compounds of formula I are inhibitors of the AChE or BChE and are therefore suitable for use in the treatment of diseases or conditions in which the level of acetylcholine is affected.

In a further aspect of the invention, the compounds of formula I are provided for use in radiotherapy. In that case, R comprises advantageously a β⁻-emitting substance, an α-emitting substance or an Auger electron-emitting substance. Therefore, compounds of formula I wherein R comprises a β⁻-emitting substance, an α-emitting substance or an Auger electron-emitting substance are used in the treatment of diseases or conditions in which the level of acetylcholine is affected. In a particular embodiment, compounds of formula I wherein R comprises a β⁻-emitting substance, such as ¹³¹I, ¹⁷⁷Lu, ⁹⁰Y, ⁸⁹Sr, ¹⁵³Sm, ¹⁸⁸Re and ¹⁸⁶Re, especially ¹³¹I, an α-emitting substance, such as ¹⁵³Sm, ²¹¹At, ²¹²Bi and ²¹²Pb, or an Auger electron-emitting substance, such as In, Gd, Pt, Ru, Os, Au, La, Ce, Ba, Cs, I, Te, Sb, Sn, Cd, Ag and Pd, especially In, Gd, Pt, Pd and Au, are used in the treatment of cancers. For example, the compound of formula RAD1 can advantageously be used in the treatment of such diseases.

When R comprises an Auger electron-emitting substance, the compound of formula I is advantageously associated with a radiation source. Preferably, the radiation source produces a photon (X- or γ-ray). Suitable radiation source comprises for example one or more radioisotope selected from ¹⁰³Pd, ^{l45}Sm, ¹⁷⁰Tm, ¹²⁵I , ¹²⁷I ²³⁴Th, ^{93m}Nb, ¹⁴⁰Ba ¹⁹⁵Au, ¹⁴⁴Ce, ^{125m}Te, ^{95m}Tc, ²⁴⁵Am, ²⁵³Eu, ¹⁸³Re, ¹⁸⁵W, ¹⁵⁹Dy, ^{127m}Te, ¹⁶⁹Yb, ¹⁰⁵ Ag, ^{119m}Sn, ¹⁷¹Tm, ¹⁴⁵Pm, ¹⁵³Gd, ¹³³Ba, ¹⁷⁴Ln, ¹⁶³ Tm, ¹⁴⁷Eu, ¹⁷⁵Hf and ^{97m}Tc. In a particular embodiment, the radiation source can be implanted near or in the body region to be treated. In another embodiment, the radiation source is introduced in the compound of formula I itself, i.e. R further comprises a radiation source as defined above.

There is also provided a compound of formula I for use in the manufucture of a medicament.

The compounds of the invention are preferably administered in a pharmaceutical composition. A pharmaceutical composition is defined in the present invention as a formulation comprising a compound of formula I in a suitable form for administration to a mammal, such as a human. The pharmaceutical composition can be formulated for anteral or parenteral administration and their preparation are well known in the art. The composition is advantageously formulated for an intravenous administration. The pharmaceutical composition comprises an effective amount of the compound of formula I with conventional carriers and excipients appropriate for the type of administration. For example, intravenous formulations advantageously contain a steril aqueous medium, such as saline, physiological serum or plasma. Such pharmaceutical composition may optionally comprise further ingredient such as buffers, pharmaceutically acceptable solubilizers, stabilizers, antioxidants or bulking agents. The pharmaceutical composition is administred in an amount wich gives a reliable image and/or therapeutic effect, taking into account the nature of the disease or condition being investigated, size of the patient, and such other factors as would be apparent to a man skilled in the art.

Therefore the invention also concerns a pharmaceutical composition comprising the compound of formula I or a pharmaceutically acceptable salt thereof. The invention further provides a pharmaceutical composition comprising a therapeutically effective amount of a compound of formula I or a pharmaceutically acceptable salt thereof together with one or more pharmaceutically acceptable adjuvant, excipient or diluent.

The following examples illustrate method for preparing the compounds of the present invention and their use as imaging agent. These examples represent specific embodiments of the invention and are not intended as limiting the scope of the invention.
Fig. 1 represents an *in vitro* autoradiographies of rat brain slices after introduction of the compound PET1.
Fig. 2 represents an *in vivo* Micro-PET imaging of rat after administration of the compound PET 1.

### Example 1: preparation of fluoroHuprine of formula PET 1

The fluoroHuprine of formula PET 1 is obtained through the fluorination of the compound HUP 13 described in EP10305366.6:

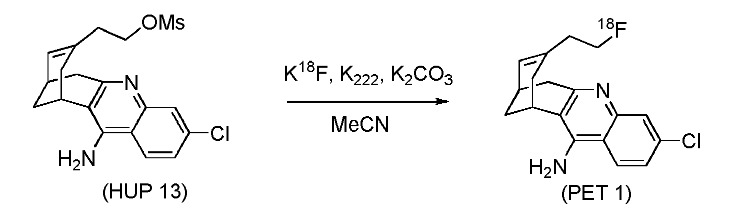

[¹⁸F]F⁻ was separated from ¹⁸O-enriched water using ion exchange resin (QMA light, Waters, ABX) eluted with a solution of potassium carbonate (2-3 mg) and Kryptofix 2.2.2 (22-24 mg) and K₂CO₃ (4.5-5.5 mg) in water (50 µL) and acetonitrile (550 µL). The water was removed azeotropically with acetonitrile (4×500 µL) under a stream of nitrogen affording a dry residue of [K/K₂₂₂]^{+ 18}F⁻.

Precursor (4-6 mg) in acetonitrile (500 µL) was added to the dried [K/K₂₂₂]^{+ 18}F⁻ complex and the sealed reaction vial was heated at 120 °C for 10 min. The crude mixture was diluted with water and loaded on a C18 cartridge conditioned with ethanol and water and dried with air. Impurities were removed by rinsing with a solution of 25% acetonitrile in 75% water (6 mL). The final product was eluted by eluting with ethanol 50% in water (2 mL).

The product is characterized by coelution with its cold ¹⁹F-fluorinated reference. Retention time: 22.90 min (ref: 21.86 min)

### Example 2: in vitro autoradiography

The compound of formula PET 1 was introduced in a composition consisting of 90 wt% physiological serum and 10 wt% ethanol so as to obtain a radiation rate of 37 MBq/mL.

The composition was incubated on brain slices of rats. Autoradiography was performed on these samples. Adult male Sprague-Dawley rats (Charles River Laboratories) were used. All animals were kept in the animal facility (room temperature 22°C with a 12 h day-night cycle). All experiments were realised in accordance with European guidelines for care of laboratory animals (86/609 EEC) and were approved by the ethical animal use committee of the laboratory.

The rat and *cat in vitro* autoradiography was performed similarly. After euthanasia by an overdose isoflurane inhalation, rat or cat brain was carefully removed and immediately frozen in 2-methylbutane cooled with dry ice (-29°C). Briefly, coronal sections (30 µm thick) cut across the hippocampus, the raphe nuclei and the cerebellum were carried out using a -20°C-cryostat (Microm-Microtech), thaw-mounted on glass slides and allowed to air dry before storage at -80°C until used. The radiotracers PET 1, the slides were allowed to reach ambient temperature and were then incubated for 20 min in Tris phosphate-buffered saline buffer (138 mM NaCl, 2.7 mM KCl, pH adjusted to 7.6) containing 37 kBq/ml (1 µCi/ml) of [¹⁸F]-PET 1.

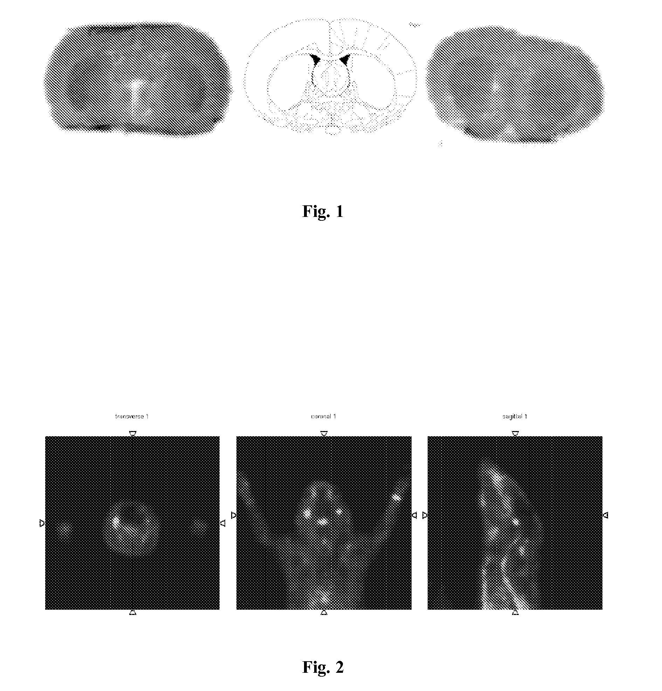

As shown in *the in vitro* radiography of Fig. 1, the striatum area appears in dark what shows that the compound can fix the striatum.

### Example 3: in vivo PET imaging

The compound of formula PET 1 was introduced in a composition consisting of 90 wt% physiological serum and 10 wt% ethanol so as to obtain a radiation rate of 37 MBq/mL.

The composition was administrated by injection in a rat. A PET imaging of the brain of the rat was preformed.

The *ex vivo and in viva* micro-PET acquisitions were realized on a camera ClearPET LYSO/LuYAP Phoswich scanner (Raytest) used in 3D mode. In the first part of the experiment, rats were anesthetized with urethane (1.25 g/kg i.p.) and an intravenous catheter was inserted in the caudal vein. PET 1 (74 MBq iv) was injected and the image is obtained on after an acquisition of 45 minutes.

*The in vivo* imaging (Fig. 2) shows that the compound of the invention can be detected in the brain of the rat. *Ex vivo* Micro-PET imaging and autoradiography of the brains of the rats also confirmed the presence of the compound PET 1 in the striatum area.

## Claims

1. A compound of formula I : 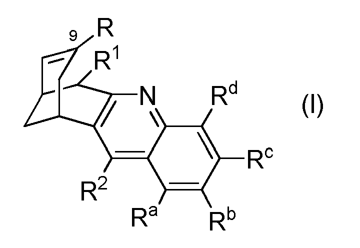 wherein,
R comprises a fluorophore or a nuclear probe;
R^{a}, R^{b}, R^{C} and R^{d} are independently H, halogen, cyano, carboxyl, -O(C₁-C₄ alkyl), ―S(C₁-C₄ alkyl), or ―CH₂S(C₁-C₄ alkyl);
R¹ is H or =CH₂; and
R² is Cl or NR³R'³ wherein R³ and R'3 are independently H, acetyl, C₁-C₄ alkyl, ―CO(C₁-C₄ alkyl) or any hydrocarbyl chain linked to a ligand of the peripheral site of the AChE or BChE;
and pharmaceutically acceptable salts thereof.

2. The compound of claim 1, wherein
R is a moiety of formula ―X―Y―Z,
wherein,
X is C₁-C₆ alkylene or C₂-C₆ alkenylene;
Y is a single bound, ―O―, ―C(O)―, ―C(O)O―, ―OC(O)―, ―NH―N=C―, ―O―N=C―, ―C=N―NH―, ―C=N―O―, ―CH(Z')―, ―CH(CH₂Z')―CH₂―, ―N(Z')―, 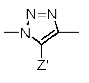 or 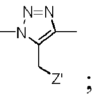
Z is a fluorophore or a nuclear probe; and
Z' is H, a lipophilic vector, a fluorophore or a nuclear probe.

3. The compound of any one of claims 1 or 2, wherein R is selected from the group consisting of ―(CH₂)₂―Z, ―(CH₂)₃―Z, ―(CH₂)₄―Z, ―(CH₂)₅―Z, ―(CH₂)₆―Z, ―(CH₂)₂―CH(Z')―Z, ―(CH₂)₂―CH(CH₂Z')―Z, 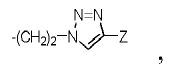 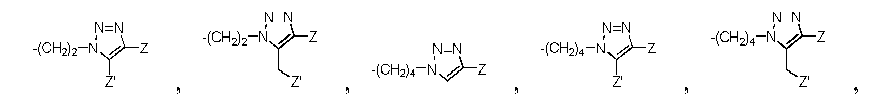 ― (CH₂)₂―NH―N=C―Z, -(CH₂)₂―O―N=C-Z, ―(CH₂)₂―C=N―NH―Z, ―(CH₂)₂―C=N―O―Z,
wherein,
Z is a fluorophore or a nuclear probe; and
Z' is a lipophilic vector, a fluorophore or a nuclear probe.

4. The compound according to any one of claims 1 to 3, wherein R² is NR ³R'³

5. The compound according to any one of claims 1 to 4, wherein R^{a}, R^{b}, R^{d} and R^{c} are H or R^{a}, R^{b} and R^{d} are H and R^{C} is Cl.

6. The compound according to any one of claims 1 to 5, wherein R¹ is H.

7. The compound according to any one of claims 1 to 6, wherein the fluorophore is selected from fluoresceine derivatives, coumarine derivatives, rhodamine derivatives and cyanine derivatives.

8. The compound according to any one of claims 1 to 6, wherein the nuclear probe is a positron-emitting radioactive substance selected from ⁶⁴Cu, ⁴⁸V, ⁵²Fe, ⁵⁵Co, ^{94m}Tc, ⁶⁸Ga ¹¹C, ¹³N , ¹⁵O, ¹⁷F , ¹⁸F, ⁶⁴Cu ⁶²CU, ⁷⁵Br, ⁷⁶Br and ¹²⁴I.

9. The compound according to any one of claims 1 to 6, wherein the nuclear probe is a gamma-emitting radioactive substance selected from ^{99m}Tc, ¹¹¹In, ^{113m}In, ⁶⁷Cu, ⁶⁷Ga, ¹¹³I, ¹²⁵I, ¹³¹I and ⁷⁷Br.

10. The compound according to any one of claims 1 to 6, wherein the nuclear probe is a paramagnetic metal ion selected from Gd (III), Mn (II), Cu (II), Cr (III), Fe (III), Co (II), Er (II), Ni (II), Eu (III) or Dy (III) or a hyperpolarised NMR-active nucleus selected from¹³C, ¹⁵N, ¹⁹F, ²⁹Si and ³¹P.

11. The compound according to any one of claims 1 to 6, wherein the nuclear probe is a β⁻-emitting radioactive substance selected from ¹³¹I, ¹⁷⁷Lu, ⁹⁰Y, ⁸⁹Sr, ¹⁵³Sm, ¹⁸⁸Re and ¹⁸⁶ Re, an α-emitting radioactive substance selected from ¹⁵³Sm, ²¹¹At, ²¹²Bi and ²¹²Pb, or an Auger electron-emitting substances selected from In, Gd, Pt, Ru, Os, Au, La, Ce, Ba, Cs, I, Te, Sb, Sn, Cd, Ag and Pd.

12. The compound according to any one of claims 1 to 6 selected from the group consisting of: 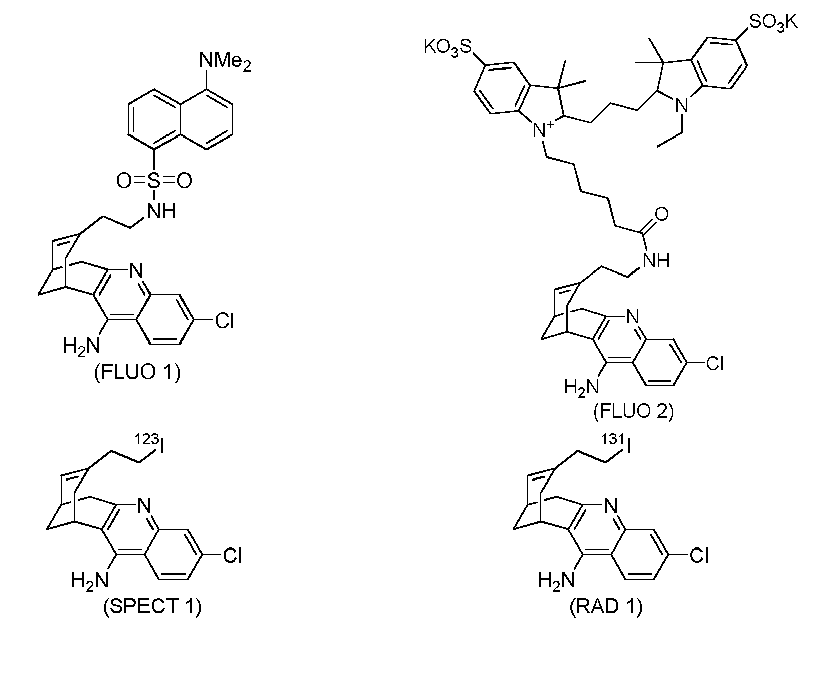 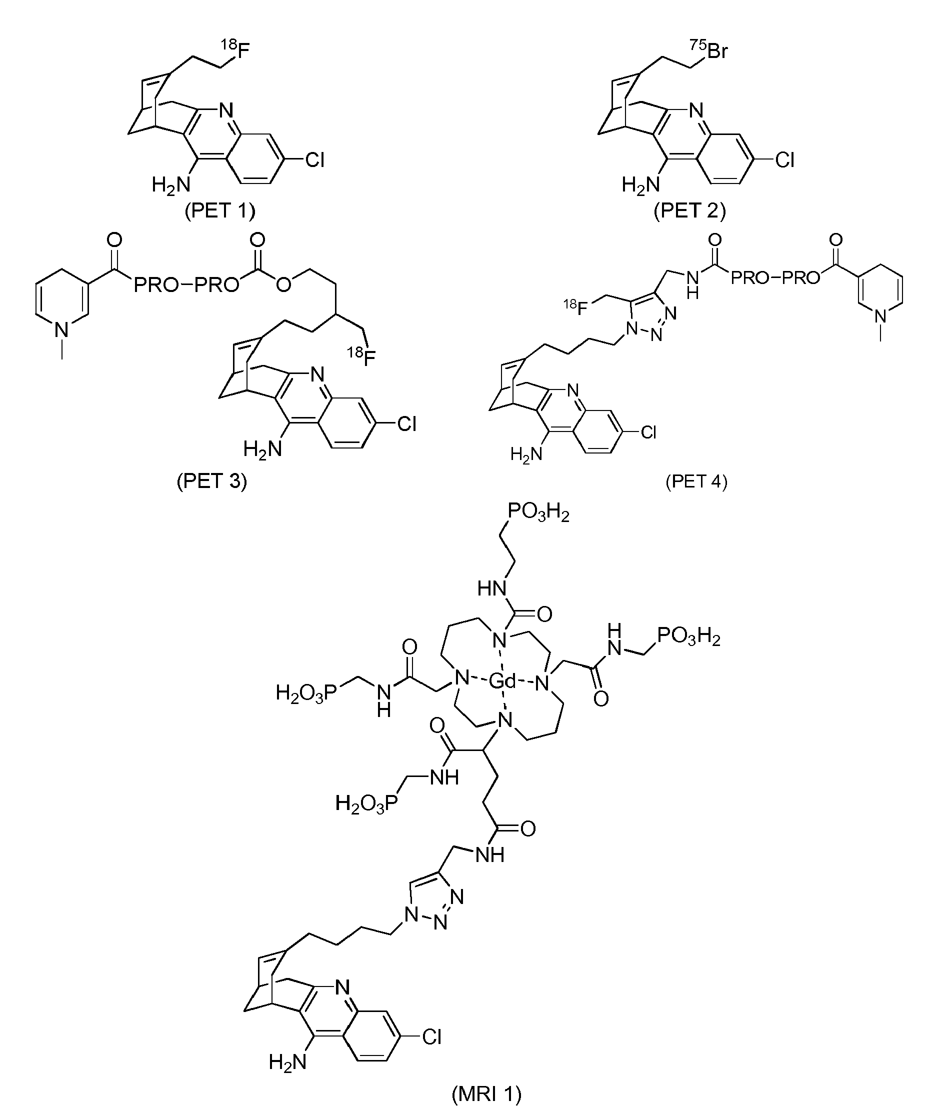 and pharmaceutically acceptable salts thereof.

13. The compound according to any one of claims 1 to 12 for use in medical imaging.

14. A method for medical imaging comprising the step of:
- administering to a patient a composition comprising the compound of claims 1 to 12;
- obtaining an image with an apparatus detecting the radiation emitted by the administered compound.

15. The compound according to any one of claims 1 to 12 for the manufacture of a medicament.

16. A composition for use in the treatment of cancer comprising the compound according to any one of claims 1 to 12 for use in the treatment of cancer.
